# EUROPÄISCHE PATENTSCHRIFT

(11) **EP 3 236 889 B1**
(45) Veröffentlichungstag und Bekanntmachung des Hinweises auf die Patenterteilung: **12.12.2018**
(21) Anmeldenummer: 15816792.4
(22) Anmeldetag: 22.12.2015
(51) Int. Cl.: A61F 5/30, A61F 5/02

(54) **PELOTTE**
PAD
PELOTE

(30) Priorität: 22.12.2014 DE 102014226841
(43) Veröffentlichungstag der Anmeldung: 01.11.2017
(73) Patentinhaber: Bauerfeind AG, 07937 Zeulenroda-Triebes (DE)
(72) Erfinder: BAUERFEIND, Hans B., 07937 Zeulenroda-Triebes (DE); SCHEUERMANN, Rainer, 24223 Raisdorf (DE)
(74) Vertreter: Wohlfahrt, Jan Günther
(86) Internationale Anmeldenummer: PCT/EP2015/080987
(87) Internationale Veröffentlichungsnummer: WO 2016/102574

(56) Entgegenhaltungen:
- EP-A1- 1 688 107
- WO-A1-2012/069923
- DE-U1-202014 100 413
- US-A- 5 388 273
- US-A1- 2003 171 703
- US-A1- 2011 095 142

## Beschreibung

Die Erfindung betrifft Pelotten für Bandagen und Orthesen. Die Erfindung stellt eine verbesserte Pelotte bereit, die eine Rückseite mit Gitternetzstruktur aufweist.

Pelotten sind medizinische Druckpolster zur Ausübung und Übertragung eines Drucks. Sie werden meist zusammen mit Bandagen oder Orthesen verwendet, beispielsweise im Rückenbereich, Hüftbereich, Schulterbereich, Armbereich oder Beinbereich.

Unterschiedliche Pelotten sind beispielsweise aus der EP 1 688 107 A1, der DE 101 03 545 A1, der DE 27 22 56 3 A1 und der DE 10 2011 010 827 A1 bekannt.

Die US 5,388,273 A stellt den nächstliegenden Stand der Technik dar und offenbart eine Rückenpelotte für eine Bandage oder Orthese, bei der die rückseitige Oberfläche zumindest in einem Teilbereich Stege aufweist, die Zwischenräume ausbilden und die vorderseitige Oberfläche Erhebungen aufweist.

Solche Pelotten können auf der Vorderseite, also der Seite, die dem Körper einer die Pelotte tragenden Person zugewandt ist und die üblicherweise an die Haut der Person angepresst ist, verschiedenste dreidimensionale Ausformungen haben, beispielsweise in Form von Noppen, Wülsten oder Luftkanälen. Die Rückseite der Pelotten ist dagegen üblicherweise glatt. Auf dieser glatten Rückseite können sich dann Befestigungselemente in Form von Druckknöpfen, Klebe- oder Klettbändern befinden, um die Pelotte an einer Bandage oder Orthese befestigen zu können. Die Pelottengrundform, also die Pelottenbasis ist ein massiver Körper, der ein entsprechendes Gewicht aufweist. Die Pelotten werden aus einem weichen und biegsamen Material hergestellt, beispielsweise Silikonkautschuk. Bei Pelotten, die eher starr am Körper eines Trägers anliegen sollen, beispielsweise bei bestimmten Rückenpelotten zum Einsatz im Lendenbereich, sind in der Pelottenbasis Verstärkungselemente integriert.

Dadurch sind die Pelotten aus dem Stand der Technik schwer. Auch kann bei den Pelotten aus dem Stand der Technik die Wirkungsintensität von vorderseitigen Erhebungen, insbesondere Noppen nur unzureichend eingestellt werden. Auch kann eine nur teilweise notwendige Versteifung der Pelotten nur durch Verwendung von Stabilisierungsplatten erreicht werden, insbesondere wenn die Pelotten an sich flexibel und weich sein sollen.

Das der Erfindung zugrunde liegende technische Problem ist die Bereitstellung einer Pelotte, deren Gewicht reduziert werden kann. Die Pelotte soll darüber hinaus einstückig herstellbar sein. Dabei soll die Pelotte auch wahlweise Bereiche mit unterschiedlicher Flexibilität beziehungsweise Steifigkeit aufweisen können.

Das der Erfindung zugrunde liegende technische Problem wird gelöst durch die Bereitstellung einer Pelotte nach Anspruch 1 sowie einer Bandage oder Orthese nach Anspruch 6.

Die Erfindung löst das ihr zugrunde liegende technische Problem insbesondere durch eine Pelotte für eine Bandage oder Orthese, wobei die Pelotte eine vorderseitige Oberfläche und eine rückseitige Oberfläche aufweist, wobei die rückseitige Oberfläche zumindest in einem Teilbereich Stege aufweist, die eine Gitternetzstruktur mit Zwischenräumen ausbilden.

Es zeigte sich überraschenderweise, dass die Ausbildung einer Gitternetzstruktur durch Stege auf der rückseitigen Oberfläche einer Pelotte nicht nur zu einer Gewichtsreduktion führt, sondern durch die Stege und ihre spezifische Ausbildung, beispielsweise die Breite der Stege und die Form und Größe der durch die Gitternetzstruktur gebildeten Zwischenräume, die Steifigkeit der Pelotte beeinflusst werden kann.

Es zeigte sich somit auch, dass durch die Ausgestaltung und Formgebung der rückseitigen Oberfläche einer Pelotte die Wirkungsweise und Wirkungsstärke der Pelotte oder bestimmte Bereiche der Pelotte beeinflusst werden kann.

Im Zusammenhang mit der vorliegenden Erfindung wird unter der vorderseitigen Oberfläche einer Pelotte die Oberfläche verstanden, die beim Tragen der Pelotte dem Körper zugewandt ist, insbesondere auf der Haut beziehungsweise Kleidung der die Pelotte verwendenden Person anliegt oder an die Haut beziehungsweise Kleidung der die Pelotte verwendenden Person angedrückt ist. Im Zusammenhang mit der vorliegenden Erfindung wird unter der rückseitigen Oberfläche einer Pelotte die der vorderseitigen Oberfläche gegenüberliegende Oberfläche verstanden, also die Oberfläche, die bei Verwendung der Pelotte dem Körper abgewandt ist. Die rückseitige Oberfläche kann insbesondere der Innenseite einer Bandage oder Orthese zugewandt sein und bevorzugt mit dieser über Verbindungselemente verbunden sein.

Die Gitternetzstruktur kann sich über einen Teil der rückseitigen Oberfläche der Pelotte erstrecken oder über die gesamte rückseitigen Oberfläche der Pelotte. Bevorzugt erstreckt sich die Gitternetzstruktur über mindestens die Hälfte der rückseitigen Oberfläche der Pelotte. In einer bevorzugten Ausführungsform erstreckt sich die Gitternetzstruktur über die gesamte rückseitige Oberfläche der Pelotte, insbesondere wenn die Pelotte keine Erhebungen auf der vorderseitigen Oberfläche aufweist.

Es werden durch die Gitternetzstruktur Zwischenräume ausgebildet, die jegliche Form und Größe haben können. Durch Anzahl, Form und Größe der Zwischenräume kann die Flexibilität und Weichheit beziehungsweise Steifigkeit der Pelotte oder wahlweise auch eines bestimmten Teilbereichs der Pelotte in vorteilhafter Weise eingestellt werden.

In einer bevorzugten Ausführungsform werden durch die Gitternetzstruktur Zwischenräume ausgebildet, die vieleckig, insbesondere viereckig, rechteckig, dreieckig, wabenförmig und rautenförmig, rund oder oval sind. Besonders bevorzugt sind die Zwischenräume rechteckig, wabenförmig oder dreieckig. Bei diesen Formen kann der Stegverlauf besonders effektiv und einfach gestaltet sein.

Eine bevorzugte rechteckige, insbesondere quadratische Form der Zwischenräume führt zu einer besonders einfachen und flexiblen Ausführungsform. Eine alternative dreieckige Form der Zwischenräume führt zu einer vorteilhaften Ausführungsform, bei der der Querverzug der Pelotte stärker eingeschränkt ist. In einer weiteren alternativen Ausführungsform können die Zwischenräume wabenförmig sein, so dass diese Ausführungsform besonders leicht ist.

Natürlich können auch verschiedene Formen der Zwischenräume kombiniert werden. Es kann auch vorgesehen sein, dass einzelne Teilbereiche der rückseitigen Oberfläche jeweils unterschiedliche Formen der Zwischenräume aufweisen.

Die Größe der Zwischenräume kann vom Fachmann nach Bedarf gewählt werden. Die Zwischenräume haben bevorzugt eine Breite und/oder eine Höhe beziehungsweise einen Durchmesser von mindestens 0,4 cm und höchstens 2 cm. Besonders bevorzugt haben die Zwischenräume eine Höhe und/oder Breite beziehungsweise einen Durchmesser von mindestens 0,7 cm und höchstens 1,5 cm.

Die Tiefe der Zwischenräume ergibt sich aus der Höhe der Stege. Im Zusammenhang mit der vorliegenden Erfindung wird die rückseitige Oberfläche der Pelotte durch die Stege gebildeten Au-ßenoberflächen gebildet und nicht durch die Böden der Zwischenräume. Somit stellen die Zwischenräume Vertiefungen in der rückseitigen Oberfläche dar.

Die Tiefe der Zwischenräume kann von einem Fachmann nach Bedarf frei gewählt werden. Die Tiefe der Zwischenräume kann beispielsweise in etwa die Hälfte der Gesamtdicke der Pelotte ausmachen, wobei sich die Gesamtdicke aus dem Abstand der vorderseitigen Oberfläche der Pelotte zu den Außenoberflächen der Stege ergibt.

Die Tiefe der Zwischenräume beträgt bevorzugt mindestens 0,2 cm und höchstens 1 cm. Besonders bevorzugt beträgt die Tiefe der Zwischenräume etwa 0,5 cm.

Dementsprechend beträgt die Höhe der Stege ebenfalls bevorzugt 0,2 cm bis 1 cm, besonders bevorzugt etwa 0,5 cm. Bevorzugt entspricht die Höhe der Stege in etwa der Hälfte der Pelottendicke.

Auch die Breite der Stege kann von einem Fachmann nach Bedarf gewählt werden. Bevorzugt beträgt die Breite der Stege mindestens 0,2 cm und höchstens 0,6 cm.

Durch die Länge, Breite und Höhe der Stege kann die Flexibilität beziehungsweise Steifigkeit der Pelotte oder des entsprechenden Pelottenbereichs in vorteilhafter Weise eingestellt werden.

Die Stege bilden durch Kreuzungspunkte die erfindungsgemäße Gitternetzstruktur. Zur Stabilisierung kann in vorteilhafter Weise vorgesehen sein, dass die Stege an den Kreuzungspunkten verbreitert sind.

Die Pelotte ist bevorzugt aus einem dauerelastischen Werkstoff, insbesondere Silikonkautschuk oder Polyurethan, bevorzugt viscoelastisches, weiches TPU, gebildet. Es können auch geschäumte, weiche Werkstoffe, beispielsweise geschäumtes, weiches TPU verwendet werden. Die Erfindung ist jedoch nicht auf diese Werkstoffe beschränkt. Der Fachmann kennt gleichermaßen geeignete Werkstoffe. Dabei ist insbesondere das physikalisch-mechanische Verhalten des Werkstoffs, vor allem der Elastizitätsmodul, an den Verwendungszweck der Pelotte angepasst.

In einer bevorzugten Ausführungsform ist die Pelotte einstückig und aus einem einzigen Material gebildet. Dies ermöglicht eine einfache und kostengünstige Herstellung. Alternativ kann aber auch vorgesehen sein, dass die Pelotte eine Stabilisierungsplatte aufweist, insbesondere eine Stabilisierungsplatte in den Pelottenkörper integriert ist.

In einer bevorzugten Ausführungsform handelt es sich bei der erfindungsgemäßen Pelotte um eine Rückenpelotte oder Hüftpelotte. Alternativ kann die Pelotte auch eine Schulterpelotte, Armpelotte, insbesondere Ellenbogenpelotte, oder eine Beinpelotte, insbesondere eine Pelotte für den Kniebereich oder den Sprunggelenksbereich, sein.

Da die erfindungsgemäße Pelotte für die Verwendung zusammen mit einer Bandage oder einer Orthese dient, wird im Zusammenhang mit der vorliegenden Erfindung unter einer Pelotte keine Schuheinlage verstanden.

In einer bevorzugten Ausführungsform weist die vorderseitige Oberfläche der Pelotte Erhebungen auf.

Im Zusammenhang mit der vorliegenden Erfindung befinden sich die Erhebungen auf der vorderseitigen Oberfläche, das heißt zur Messung der Dicke der Pelotte wird die Höhe der Erhebungen nicht mit einberechnet.

Die Erhebungen können jegliche Formen haben, beispielsweise wulstförmig, kegelförmig, oder noppenförmig, insbesondere halbkugelförmig, sein.

In einer bevorzugten Ausführungsform sind die Erhebungen noppenförmig, also Noppen.

Beispielsweise kann es sich bei den Noppen um Friktionsnoppen handeln, die beim Tragen der Pelotte durch Druck und Reibung zu einem Massageeffekt führen.

In einer bevorzugten Ausführungsform sind die Erhebungen, insbesondere die Noppen, aus dem gleichen Material, wie die Pelotte gebildet. Eine einstückige Pelotte umfasst somit auch die Erhebungen, insbesondere Noppen.

Die Erhebungen, insbesondere Noppen, einer Pelotte sollen an spezifischen Stellen einen besonders starken Druck beim Tragen der Pelotte ausüben. Daher ist es vorteilhaft, wenn die Pelotte im Bereich der Erhebungen, insbesondere Noppen, härter ist. Dagegen soll die Pelotte in den Bereichen, in denen sich keine Erhebungen, insbesondere Noppen, befinden nicht so hart sein, sondern sollte eher weich sein, da dies zu einem bequemeren Tragegefühl führt. Die erfindungsgemäßen Gitternetzstrukturen können in vorteilhafter Weise für weichere Bereiche der Pelotte eingesetzt werden, während härtere Bereiche für die Noppen durch ein dortiges Weglassen der Gitternetzstruktur erzeugt werden können. Dies bedeutet, dass in einer bevorzugten Ausführungsform die Bereiche der rückseitigen Oberfläche, die den Erhebungen, insbesondere Noppen, gegenüberliegen, keine Gitternetzstruktur aufweisen, also es sich dort keine Stege mit Zwischenräumen befinden, sondern dortige Zwischenräume mit dem Pelottengrundmaterial ausgefüllt sind. Dadurch kann eine Pelotte erhalten werden, die durch die Gitternetzstruktur im Grundaufbau leicht und flexibel ist, jedoch im Bereich der Erhebungen, insbesondere Noppen, steifer und härter ist. Somit ist es auch in vorteilhafter Weise möglich, dass auf eine Stabilisierungsplatte im Inneren der Pelotte verzichtet werden kann und die Pelotte einstückig ausgebildet werden kann. Dadurch, dass bei einer erfindungsgemäßen Pelotte, insbesondere bei einer Rückenpelotte, auf eine Stabilisierungsplatte verzichtet werden kann, kann sich die erfindungsgemäße Pelotte in vorteilhafter Weise der anatomischen Oberfläche des anliegenden Körpers anpassen und es kann ein gleichmäßiger Wechseldruck zustande kommen. Bei zusätzlichem Bedarf kann jedoch auch eine Stabilisierungsplatte vorhanden sein.

Durch die Wahl, ob im Bereich einzelner Erhebungen, insbesondere Noppen, die rückeiteige Oberfläche eine Gitterstruktur aufweist oder nicht kann in vorteilhafter Weise bestimmt werden ob die jeweilige Erhebung, insbesondere Noppe, tiefer oder weniger tief in die beim Verwenden der Pelotte anliegenden Weichteile des Körpers eindringen.

In einer bevorzugten Ausführungsform sind somit in den Bereichen der rückseitigen Oberfläche, die den Bereichen der vorderseitigen Oberfläche, an denen sich die Erhebungen, insbesondere Noppen, befinden, gegenüber liegen, keine Zwischenräume ausgebildet. Es kann aber auch vorgesehen sein, dass nur in manchen dieser Bereiche der rückseitigen Oberfläche, die den Bereichen der vorderseitigen Oberfläche, an denen sich die Erhebungen, insbesondere Noppen, befinden, gegenüber liegen, keine Zwischenräume ausgebildet sind.

Die Pelotte kann auf der rückseitigen Oberfläche Befestigungselemente zum Befestigen der Pelotte auf der Innenseite einer Bandage oder Orthese aufweisen. Solche Befestigungselemente können beispielsweise Druckknöpfe, Hackenmechanismen, ein Klebestreifen oder ein Klettstreifen sein.

In einer bevorzugten Ausführungsform weist eine erfindungsgemäße Rückenpelotte mit insgesamt sieben bis elf Noppen, bevorzugt acht Noppen, auf, wobei die Noppen drei waagerechte Reihen bilden, wobei die obere Reihe zwei bis drei, bevorzugt zwei, die mittlere Reihe drei bis fünf, bevorzugt vier, und die untere Reihe zwei bis drei, bevorzugt zwei Noppen aufweist.

Bevorzugt haben die Noppen dabei einen Durchmesser von etwa mindestens 1 cm bis höchstens 2,5 cm, besonders bevorzugt von mindestens 1,5 cm bis höchstens 2 cm. Bevorzugt haben die in einer Reihe liegenden Noppen einen Abstand von mindestens 3 cm und höchstens 9 cm. Bevorzugt haben dabei die Noppen einen Abstand zu den jeweils benachbarten Noppen von mindestens 3 cm und höchstens 9 cm.

Die vorliegende Erfindung betrifft auch eine Bandage oder Orthese enthaltend eine erfindungsgemäße Pelotte.

In einer bevorzugten Ausführungsform handelt es sich um eine Rückenbandage oder eine Rückenorthese. Diese kann besonders als Gewirkorthese, Gestrickorthese, Gewirkbandage oder Gestrickbandage mit eingelegter erfindungsgemäßer Pelotte ausgestaltet sein. Alternativ kann es sich auch um eine andere Bandage oder Orthese handeln, insbesondere um eine Fußbandage, Fußorthese, Kniebandage, Knieorthese, Oberschenkelbandage, Oberschenkelorthese, Hüftbandage, Hüftorthese, Schulterbandage, Schulterorthese, Ellenbogenbandage, Ellenbogenorthese und Handbandage oder Handorthese handeln.

Bei einer erfindungsgemäßen Bandage oder Orthese ist die erfindungsgemäße Pelotte bevorzugt mit der rückseitigen Oberfläche an der Innenseite der Bandage oder Orthese befestigt, insbesondere über mindestens ein Befestigungselement befestigt. Positionierungs- und Befestigungsmöglichkeiten sind einem Fachmann bekannt.

Gegenstand der Erfindung ist auch die therapeutische und/oder prophylaktische Verwendung der erfindungsgemäßen Pelotte bei der Behandlung des Lendenwirbelsäulensyndroms durch Druckmassage. Insbesondere kann die Pelotte dabei zur Triggerpunktmassage, zur Friktionsmassage, zur Wechseldruckmassage, zur intermittierenden Kompression, zur Detonisation und/oder zur Oberflächenstimmulation der Haut verwendet werden.

Die Erfindung wird anhand der nachfolgenden Figuren näher beschrieben, ohne dass die dort dargestellten Ausführungsformen der Erfindung beschränkend zu verstehen sind.
Figur 1 zeigt die vorderseitige Oberfläche einer bevorzugten Ausführungsform der erfindungsgemäßen Pelotte;
Figur 2 zeigt eine bevorzugte Ausführungsform der rückseitigen Oberfläche einer erfindungsgemäßen Pelotte;
Figur 3 zeigt eine alternative Ausführungsform der rückseitigen Oberfläche einer erfindungsgemäßen Pelotte;
Figur 4 zeigt eine erfindungsgemäße Pelotte zusammen mit einer Bandage;
Figur 5 zeigt eine weitere alternative Ausführungsform der rückseitigen Oberfläche einer erfindungsgemäßen Pelotte.

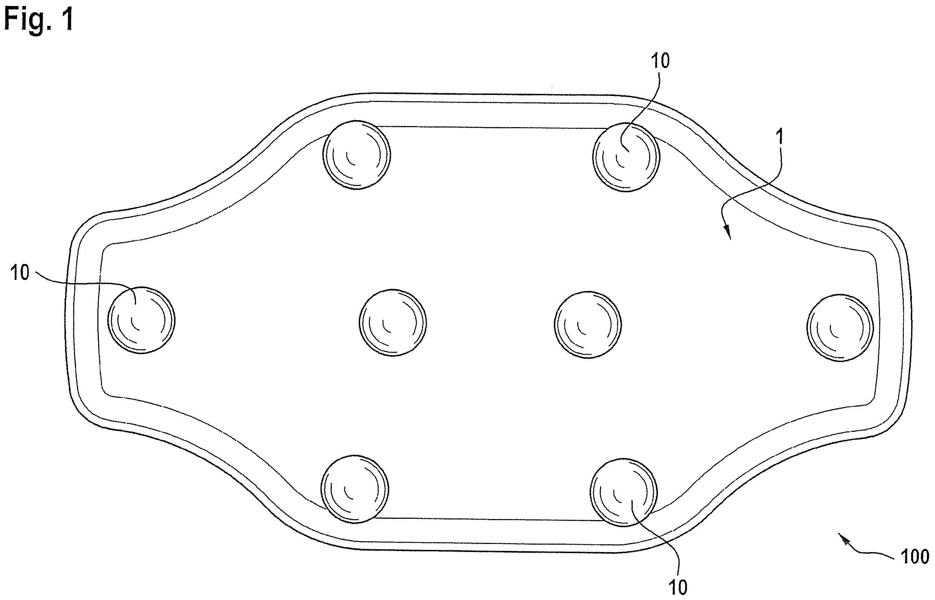

Figur 1 zeigt die vorderseitige Oberfläche 1 einer erfindungsgemäßen Pelotte 100. Die Pelotte 100 ist hier eine Rückenpelotte. Die Pelotte 100 ist einstückig aus einem weichen und flexiblen Material gefertigt. Die Oberfläche 1 ist an den Kanten abgerundet. Auf der Oberfläche 1 befinden sich halbkugelförmige Friktionsnoppen 10. Deren Anzahl und Positionierung führt in vorteilhafter Weise zu einer Stimulierung der Triggerpunkte durch Friktionsmassage im Rückenbereich.

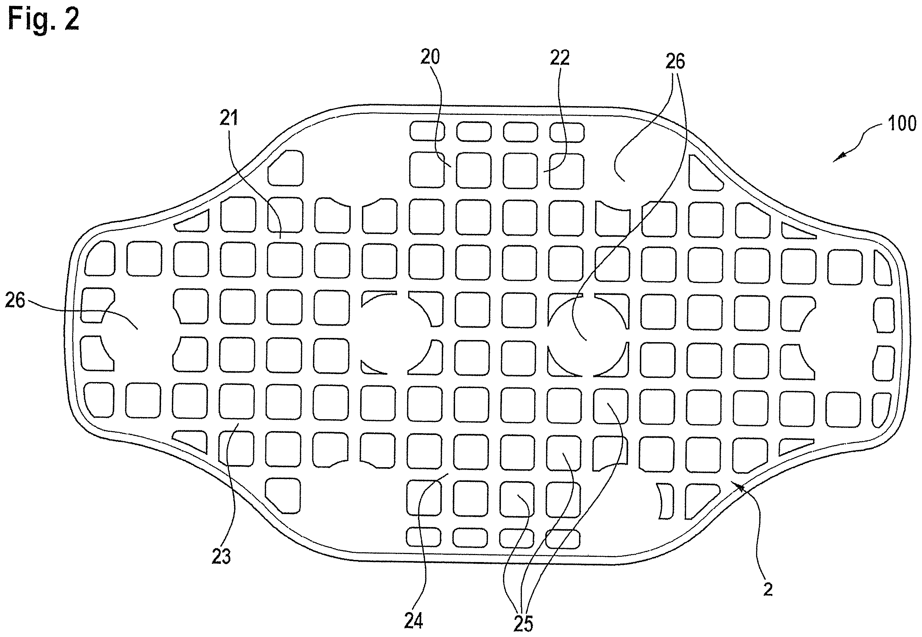

Figur 2 zeigt die rückseitige Oberfläche 2 der Rückenpelotte 100. Die Oberfläche 2 wird aus den Außenoberflächen einer Vielzahl von Stegen 20, 21, 22, 23 gebildet, die durch Kreuzungspunkte 24 eine Gitternetzstruktur mit Zwischenräumen 25 ausbilden. Die Zwischenräume 25 sind somit Vertiefungen in der rückseitigen Oberfläche 2. Im Bereich der Kreuzungspunkte 24 sind die Stege 20, 21, 22, 23 zur Stabilisierung etwas verstärkt. In dem Bereich der rückseitigen Oberfläche 2, bei dem gegenüberliegend auf der vorderseitigen Oberfläche 1 der Pelotte 100 Noppen 10 vorhanden sind, befindet sich keine Gitterstruktur mit Zwischenräumen. Vielmehr sind die Zwischenräume auf die Höhe der Stege 20, 21, 22, 23 aufgefüllt und bilden somit härte Bereiche 26 aus, wodurch die Noppen bei Verwendung der Pelotte mit einer Bandage oder Orthese durch die Bandage oder Orthese besonders gut und kräftig auf die Haut angedrückt werden.

In der gezeigten Ausführungsform bilden die Stege 20, 21, 22, 23 quadratische Vertiefungen 25, wobei die Ecken der Vertiefungen 25 durch die verdickten Kreuzungspunkte 24 abgerundet sind.

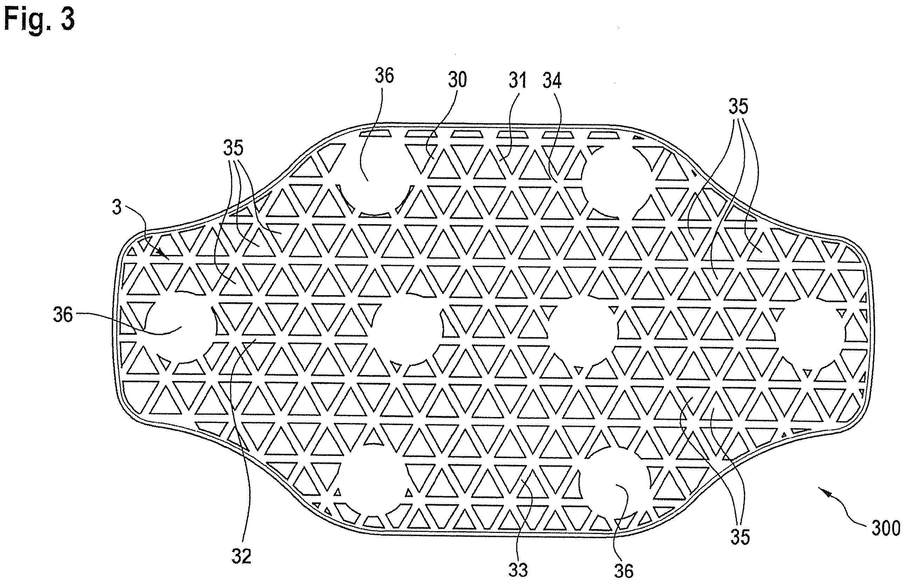

Figur 3 zeigt eine alternative Ausführungsform einer rückseitigen Oberfläche 3 einer erfindungsgemäßen Pelotte 300. Auch diese Ausführungsform weist wieder eine Vielzahl von Stegen 30, 31, 32, 33 auf, die durch die Kreuzungspunkte 34 eine Gitternetzstruktur mit Zwischenräumen 35 bilden. Auch sind wieder im Bereich der gegenüberliegenden Noppen Verstärkungspunkte 36 vorhanden.

Im Vergleich zu der rechteckigen Gitternetzstruktur der Ausführungsform von Figur 2 bilden die hier gezeigten Stege 30, 31, 32, 33 eine Gitternetzstruktur mit dreieckigen Zwischenräumen 35 aus. Durch diese Struktur wird der Querverzug der Pelotte 300 stärker eingeschränkt.

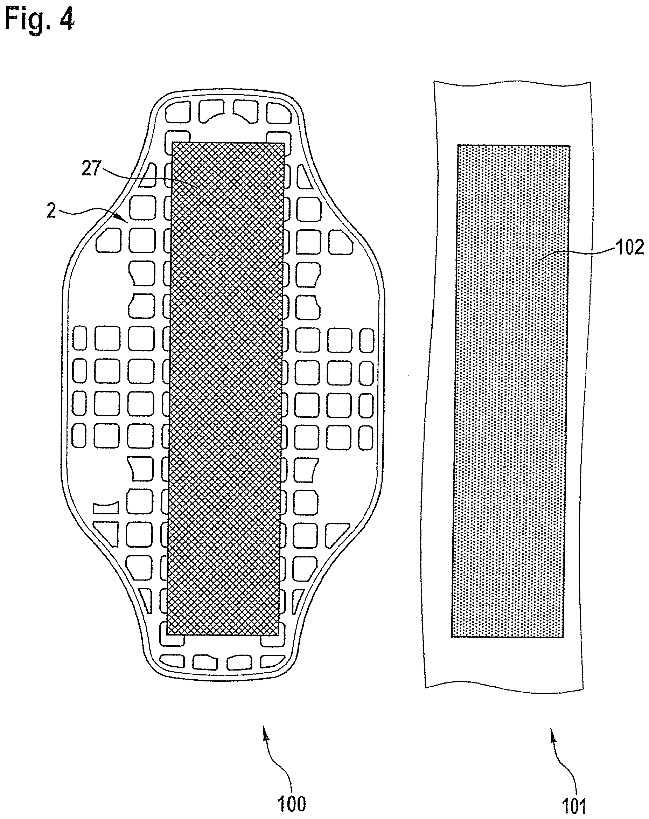

Figur 4 zeigt eine erfindungsgemäße Pelotte 100 zusammen mit einem Teilstück einer Bandage 101. Die rückseitige Oberfläche 2 der Pelotte 100 weist ein Klettband 27 auf, mit dem die Pelotte 100 an ein Flauschband 102 der Bandage 101 befestigt werden kann.

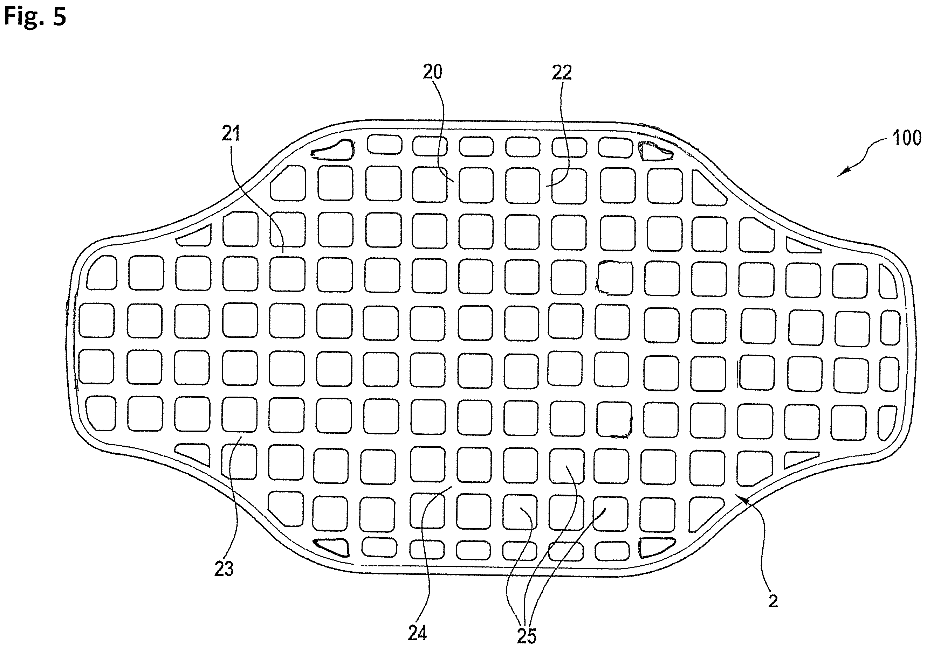

Figur 5 zeigt eine weitere alternative Ausführungsform der rückseitigen Oberfläche 2 einer erfindungsgemäßen Pelotte 100. Der Aufbau ist grundsätzlich wie in Figur 2. In der gezeigten Ausführungsform bilden wieder die Stege 20, 21, 22, 23 quadratische Vertiefungen 25, wobei die Ecken der Vertiefungen 25 durch die verdickten Kreuzungspunkte 24 abgerundet sind. In dem Bereich der rückseitigen Oberfläche 2, bei dem gegenüberliegend auf der vorderseitigen Oberfläche 1 der Pelotte 100 Noppen 10 vorhanden sind, befindet sich hier aber auch eine Gitterstruktur mit Zwischenräumen. Somit sind hier keine härten Bereiche vorhanden. Dies führt zu einer weicheren Pelotte als die aus Figur 2.

## Patentansprüche

1. Rückenpelotte (100, 300) für eine Bandage (101) oder Orthese, wobei die Rückenpelotte (100) eine vorderseitige Oberfläche (1) und eine rückseitige Oberfläche (2) aufweist, wobei die rückseitige Oberfläche (2,3) zumindest in einem Teilbereich Stege (20,21,22,23,30,31,32,33) aufweist, wobei die vorderseitige Oberfläche (1) Erhebungen (10) aufweist, **dadurch gekennzeichnet, dass** die Rückenpelotte (100,300) einstückig und aus einem einzigen Material gebildet ist, und dass die Stege (20,21,22,23,30,31,32,33) eine Gitternetzstruktur mit Zwischenräumen (25,35) ausbilden.

2. Rückenpelotte nach Anspruch 1, wobei durch die Gitternetzstruktur Zwischenräume (25,35) ausgebildet werden, die rechteckig, dreieckig, wabenförmig, rautenförmig rund oder oval sind.

3. Rückenpelotte nach einem der vorhergehenden Ansprüche, wobei sich die Gitternetzstruktur über die gesamte rückseitige Oberfläche (2,3) der Rückenpelotte (100, 300) erstreckt.

4. Rückenpelotte nach einem der vorhergehenden Ansprüche, wobei die Erhebungen (10) noppenförmig sind.

5. Rückenpelotte nach einem der vorhergehenden Ansprüche, wobei in den Bereichen der rückseitigen Oberfläche (2,3), die den Bereichen der vorderseitigen Oberfläche (1), an denen sich die Erhebungen (10) befinden, gegenüber liegen, keine Zwischenräume ausgebildet sind.

6. Rückenbandage (101) oder Rückenorthese enthaltend eine Rückenpelotte (100,300) nach einem der vorhergehenden Ansprüche.

## Claims

1. A back pad (100, 300) for a bandage (101) or orthosis, wherein the back pad (100) has a front surface (1) and a rear surface (2), wherein at least a subarea of the rear surface (2, 3) has connection pieces (20, 21, 22, 23, 30, 31, 32, 33), wherein the front surface (1) has projections (10), **characterised in that** the back pad (100, 300) is one piece and made of a single material and that the connection pieces (20, 21, 22, 23, 30, 31, 32, 33) form a mesh structure having interstices (25, 35).

2. The back pad according to claim 1, wherein interstices (25, 35) that are formed by the mesh structure, are rectangular, triangular, honeycomb shaped, diamond shaped, round or oval.

3. The back pad according to any of the preceding claims, wherein the mesh structure extends across the entire rear surface (2, 3) of the back pad (100, 300).

4. The back pad according to any of the preceding claims, wherein the projections (10) are knob shaped.

5. The back pad according to any of the preceding claims, wherein no interstices are formed in the areas of the rear surface (2,3) that are opposite the areas of front surface (1) on which projections (10) are located.

6. A back bandage (101) or orthosis containing a back pad (100, 300) according to any of the preceding claims.

## Revendications

1. Pelote dorsale (100, 300) pour un bandage (101) ou une orthèse, la pelote dorsale (100) présentant une surface avant (1) et une surface arrière (2), la surface arrière (2, 3) présentant des nervures (20, 21, 22, 23, 30, 31, 32, 33) au moins dans une zone partielle, la surface avant (1) présentant des élévations (10), **caractérisée en ce que** la pelote dorsale (100, 300) est réalisée d'une seule pièce et dans une seule matière, et **en ce que** les nervures (20, 21, 22, 23, 30, 31, 32, 33) forment une structure quadrillée munie d'espaces intermédiaires (25, 35).

2. Pelote dorsale selon la revendication 1, dans laquelle des espaces intermédiaires (25, 35) sont formés par la structure quadrillée, lesquels sont rectangulaires, triangulaires, alvéolaires, en forme de losange, ronds ou ovales.

3. Pelote dorsale selon l'une quelconque des revendications précédentes, dans laquelle la structure quadrillée s'étend sur toute la surface arrière (2, 3) de la pelote dorsale (100, 300).

4. Pelote dorsale selon l'une quelconque des revendications précédentes, dans laquelle les élévations (10) sont en forme de nopes.

5. Pelote dorsale selon l'une quelconque des revendications précédentes, dans laquelle, dans les zones de la surface arrière (2, 3) qui sont opposées aux zones de la surface avant (1), sur lesquelles se trouvent les élévations (10), aucun espace intermédiaire n'est réalisé.

6. Bandage dorsal (101) ou orthèse dorsale comprenant une pelote dorsale (100, 300) selon l'une quelconque des revendications précédentes.
